(19) Europäisches Patentamt
European Patent Office
Office Européen des brevets

(11) Publication number: 0 240 047 B1

# (12) EUROPEAN PATENT SPECIFICATION

(45) Date of publication of patent specification: 02.01.91 Bulletin 91/01

(51) Int. Cl.[5]: C07D 235/02, A01N 43/52

(21) Application number: 87200351.2

(22) Date of filing: 26.02.87

## (54) Fungicides.

(30) Priority: 28.02.86 GB 8605032

(43) Date of publication of application: 07.10.87 Bulletin 87/41

(45) Publication of the grant of the patent: 02.01.91 Bulletin 91/01

(84) Designated Contracting States: AT BE CH DE ES FR GB GR IT LI LU NL SE

(56) References cited:
CHEMISCHE BERICHTE, vol. 103, no. 10, 1970, pages 3104-3113, Verlag Chemie GmbH, Weinheim, DE; L. CAPUANO et al.: "Heterocyclisierungen, VIII: Ungewöhnliche Bildung von 3-Phenyl-naphth[1.2-d]imidazoldionen-(2.5)
ANNALEN DER CHEMIE, nr. 12, 1976, pages 2222-2239, Weinheim, DE; A. HEESING et al.: "Synthese und polarographisches Verhalten von offenkettigen sowie heterocyclischen chinoiden Guanidin- und Harnstoffderivaten"

(73) Proprietor: SHELL INTERNATIONALE RESEARCH MAATSCHAPPIJ B.V.
Carel van Bylandtlaan 30
NL-2596 HR Den Haag (NL)

(72) Inventor: Entwistle, Ian David
44 Woodside Gardens
Sittingbourne Kent (GB)
Inventor: Harkin, Shaun Anthony
61 Sandford Road
Sittingbourne Kent (GB)
Inventor: Slight, Peter John
44 Vectis Drive
Sittingbourne Kent (GB)

(74) Representative: Bennett, David Arthur Horder et al
4, York Road
London SE1 7NA (GB)

EP 0 240 047 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a composition for and a method of combating fungus, and to compounds for use in such a composition or method

Ann. Chem. (1976) 2222-39 describes the synthesis and polarographic properties of certain naphthimidazolones of structure :

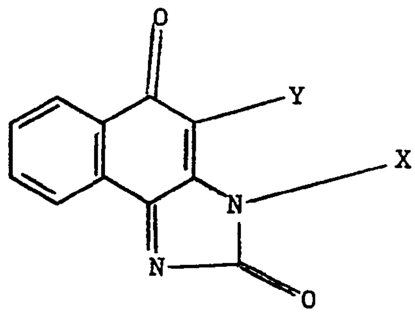

I

wherein X is a hydrogen atom or phenyl group and Y is a chlorine atom or X is a phenyl group and Y is a bromine atom. There is no teaching in that reference, nor elsewhere in the prior art, that compounds of this structural type have fungicidal activity.

It has now been discovered that a group of naphthimidazolones, some of which are novel, has useful fungicidal activity. Accordingly, the present invention provides a fungicidal composition which comprises a carrier and, as active ingredient, a naphthimidazolone of the formula II :

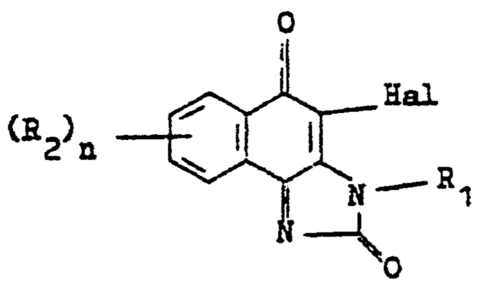

II

wherein Hal represents a halogen, preferably fluorine, chlorine or bromine, atom ; $R_1$ represents a hydrogen atom or an optionally substituted alkyl, cycloalkyl, alkenyl, alkynyl, aralkyl or aryl, preferably phenyl, group; $R_2$ represents a halogen, preferably fluorine or chlorine, atom or an alkyl or haloalkyl group ; and n is 0, 1 or 2.

When any of the foregoing substituents represents or contains an alkyl, alkenyl or alkynyl substituent group, this may be linear or branched and may contain up to 14, preferably up to 6, and especially up to 4, carbon atoms, suitable examples being methyl, ethyl, propyl, butyl, tridecyl, propenyl and propynyl. When they represent a cycloalkyl group this may contain from 3 to 10, preferably 3 to 8, carbon atoms, and is suitably cyclopropyl or cyclohexyl. When they contain an aralkyl or aryl substituent group, this is preferably a benzyl or phenyl group respectively. When any of the foregoing substituents are designated as being optionally substituted, the substituent groups which are optionally present may be any of those customarily employed in the development of pesticidal compounds, and/or the modification of such compounds to influence their structure/activity, persistence, penetration or other property. Specific examples of such substituents include halogen, especially fluorine, chlorine or bromine, atoms, and nitro, cyano, hydroxyl, alkyl, haloalkyl, alkoxy and alkoxy carbonyl groups.

Preferably Hal represents a fluorine or, more especially, a chlorine atom ; $R_1$ represents a hydrogen atom, an alkyl group of 1 to 14 carbon atoms, especially methyl, ethyl, propyl, butyl or tridecyl, optionally substituted by a fluorine or chlorine atom, or a cyano, alkoxy or alkoxycarbonyl group, an alkenyl or alkynyl group of up to 6 carbon atoms, especially propenyl or propynyl, a cycloalkyl group of 3 to 8 carbon atoms, especially cyclopropyl or cyclohexyl, optionally substituted by a fluorine or chlorine atom, a benzyl group or a phenyl group ; $R_2$ represents a chlorine atom, or an alkyl group of 1 to 6 carbon atoms, especially methyl,

and n is 0 or 1. In particular, the more interesting levels of fungicidal activity appear to be found in those compounds of formula II wherein Hal represents a chlorine atom ; $R_1$ represents an alkyl group of 1 to 4 carbon atoms optionally substituted by a fluorine or chlorine atom or a cyano, methoxy or ethoxycarbonyl group ; $R_2$ represents a chlorine atom and n is 0 or 1

Many of the compounds of formula II are novel and the invention therefore also extends to these novel compounds per se. The novel compounds are the naphthimidazolones of formula II wherein the substituents are as defined above, provided that, when Hal is a chlorine atom and n=0, then $R_1$ is not a hydrogen atom or a phenyl group, and, when Hal is a bromine atom and n=0, then $R_1$ is not a phenyl group.

The invention also provides a process for the preparation of compounds of the general formula II, which comprises reacting an amino naphthoquinone of formula III :

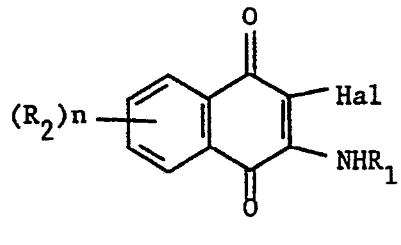

III

with chlorosulphonylisocyanate ($ClSO_2NCO$), the terms Hal, $R_1$, $R_2$ and n having the meanings defined above. The reaction is suitably carried out by warming together the reactants, e.g. to a temperature of about 50°C, and the desired product is conveniently recovered by adding the mixture to ice, followed by filtration

The invention includes also a method for making a fungicidal composition as defined above which comprises bringing a compound of formula II into association with at least one carrier.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating fungicidal compositions may be used. Preferably compositions according to the invention contain 0.5 to 95% by weight of active ingredient.

Suitable solid carriers include natural and synthetic clays and silicates, for example natural silicas such as diatomaceous earths ; magnesium silicates, for example talcs ; magnesium aluminium silicates, for example attapulgites and vermiculites ; aluminium silicates, for example kaolinites, montmorillonites and micas ; calcium carbonate ; calcium sulphate ; ammonium sulphate ; synthetic hydrated silicon oxides and synthetic calcium or aluminium silicates ; elements, for example carbon and sulphur ; natural and synthetic resins, for example coumarone resins, polyvinyl chloride, and styrene polymers and copolymers ; solid polychlorophenols ; bitumen ; waxes ; and solid fertilisers, for example superphosphates.

Suitable liquid carriers include water ; alcohols, for example isopropanol and glycols ; ketones, for example acetone, methyl ethyl ketone, methyl isobutyl ketone and cyclohexanone ; ethers ; aromatic or araliphatic hydrocarbons, for example benzene, toluene and xylene ; petroleum fractions, for example kerosine and light mineral oils; chlorinated hydrocarbons, for example carbon tetrachloride, perchloroethylene and trichloro-ethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent ; it may be nonionic or ionic. Examples of suitable surface-active agents include the sodium or calcium salts of polyacrylic acids and lignin sulphonic acids ; the condensation of fatty acids or aliphatic amines or amides containing at least 12 carbon atoms in the molecule with ethylene oxide and/or propylene oxide ; fatty acid esters of glycerol, sorbitol, sucrose or pentaerythritol ; condensates of these with ethylene oxide and/or propylene oxide ; condensation products of fatty alcohol or alkyl phenols, for example p-octylphenol or p-octylcresol, with ethylene oxide and/or propylene oxide ; sulphates or sulphonates of these condensation products ; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate ; and polymers of ethylene oxide and copolymers of ethylene oxide and propylene

oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing $\frac{1}{2}$-10% w of active ingredient. Granules are usually prepared to have a size between 10 and 100 BS mesh (1.676 -0.152 mm), and may be manufactured by agglomeration or impregnation techniques. Generally, granules will contain $\frac{1}{2}$-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble ; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The compositions may also contain other ingredients, for example other compounds possessing pesticidal, especially insecticidal, acaricidal, herbicidal or fungicidal, properties.

Of particular interest in enhancing the duration of the protectant activity of the compounds of this invention is the use of a carrier which will provide a slow release of the fungicidal compounds into the environment of the plant which is to be protected. Such slow-release formulations could, for example, be inserted in the soil adjacent to the roots of a vine plant, or could include an adhesive component enabling them to be applied directly to the stem of a vine plant.

The invention still further provides the use as a fungicide of a naphthimidazolone of the general formula II as defined above, and a method for combating fungus at a locus, which comprises treating the locus, which may for example be plants subject to or subjected to fungal attack, seeds of such plants or the medium in which such plants are growing or are to be grown, with such a derivative.

The present invention is of wide applicability in the protection of crop plants against fungal attack. Typical crops which may be protected include vines, grain crops such as wheat and barely, rice, beans and apples. The duration of protection is normally dependent on the individual compound selected, and also a variety of external factors, such as climate, whose impact is normally mitigated by the use of a suitable formulation.

The invention will be further understood from the following Examples.

## Example 1

### 3-Methyl-4-chloronaptho-(1,2-d)-imidazole-2,5-dione

Chlorosulphonyl isocyanate (595 ml) was added dropwise over 2 hours to an ice-cooled suspension of 2-chloro-3-methylamino-1,4-naphthoquinone (1.4 kg) in methylene dichloride (7.5 litres). The reaction mixture was stirred for a further 2 hours at ice temperature, and was then poured cautiously into excess water. The organic phase was separated and the aqueous phase re-extracted with methylene dichloride. The combined organic extracts were dried (magnesium sulphate), filtered and evaporated to reduced volume. The resulting red solution was passed through a short silica column to yield the desired product as a red solid, m.pt. (with decomp.) above 215°C.

Analysis

Calc. for $C_{12}H_7N_2O_2Cl$ : C 58.4 ; H 2.9 ; N 11.4
Found : C 58.2 ; H 3.1 ; N 11.6

Examples 2-34

Following a procedure similar to that described in Example 1, further 4-chloronaphimidazolone derivatives were prepared, whose physical characteristics and analyses are given in Table 1 below. In this Table, the compounds are identified by reference to the substituents $R_1$ and $R_2$ in the formula :

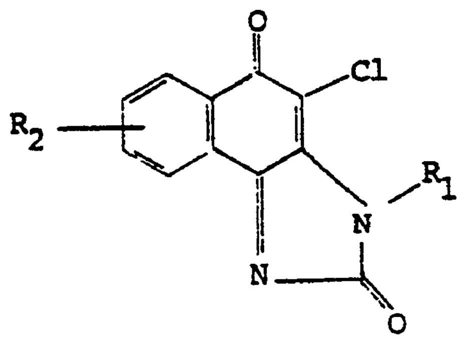

TABLE 1

| Ex. No. | $R_1$ | $R_2$ | M.Pt. °C | Analysis |
|---|---|---|---|---|
| 2 | H | H | >215 | Calc. C 56.7; H 2.1; N 12.0; Cl 15.2<br>Found C 56.1; H 3.1; N 11.4; Cl 15.0 |
| 3 | $C_2H_5$ | H | 170-3 | Calc. C 59.9; H 3.5; N 10.7<br>Found C 58.4; H 3.6; N 10.5 |
| 4 | $CH(CH_3)_2$ | H | 201-2 (dec) | Calc. C 61.2; H 4.0; N 10.2; Cl 13.0<br>Found C 62.1; H 4.1; N 10.2; Cl 13.1 |
| 5 | $CH_2CH(CH_3)_2$ | H | 161-4 | Calc. C 62.4; H 4.5; N 9.7<br>Found C 60.8; H 4.4; N 9.7 |
| 6 | $C_{13}H_{27}$ | H | 118-122 | Calc. C 69.5; H 7.5; N 6.7<br>Found C 68.9; H 7.4; N 7.6 |
| 7 | $CH_2$Phenyl | H | 212-4 | Calc. C 67.0; H 3.4; N 8.7<br>Found C 65.7; H 3.5; N 8.6 |

TABLE 1 continued...

| Ex. No. | $R_1$ | $R_2$ | M.Pt. °C | Analysis |
|---|---|---|---|---|
| 8 | Cyclohexyl | H | 261-3 (dec) | Calc. C 64.9; H 4.8; N 8.9<br>Found C 64.1; H 4.8; N 9.2 |
| 9 | $CH_2CH=CH_2$ | H | 159-161 | Calc. C 61.7; H 3.3; N 10.3<br>Found C 60.8; H 3.6; N 10.6 |
| 10 | $CH_2C \equiv CH$ | H | 220-2 (dec) | Calc. C 62.1; H 2.6; N 10.3<br>Found C 62.1; H 2.7; N 10.4 |
| 11 | Phenyl | H | 255 | Calc. C 66.3; H 2.3; N 9.1; Cl 11.5<br>Found |
| 12 | $CH_2CF_3$ | H | 227-231 | Calc. C 49.6; H 1.9; N 8.9<br>Found C 49.0; H 2.3; N 9.5 |

TABLE 1 continued...

| Ex. No. | $R_1$ | $R_2$ | M.Pt. °C | Analysis |
|---|---|---|---|---|
| 13 | $CH_2CF_3$ | Cl | 239 | Calc. C 44.7; H 1.4; N 8.0<br>Found C 44.6; H 1.9; N 8.6 |
| 14 | $CH_2CH_2Cl$ | H | 191-3 | Calc. C 52.9; H 2.7; N 9.5<br>Found C 51.4; H 3.0; N 9.7 |
| 15 | $CH_2CH_2Cl$ | Cl | 209-11 | Calc. C 47.4; H 2.1; N 8.5<br>Found C 47.3; H 2.4; N 8.9 |
| 16 | $CH_2CH_2CH_2Cl$ | H | 178-182 | Calc. C 54.4; H 3.3; N 9.1<br>Found C 54.0; H 3.3; N 9.1 |
| 17 | $CH_2CH_2CH_2Cl$ | Cl | 215-226 | Calc. C 48.9: H 2.6; N 8.2<br>Found C 49.0; H 2.7; N 8.4 |

**TABLE 1 continued...**

| Ex. No. | $R_1$ | $R_2$ | M.Pt.°C | Analysis |
|---|---|---|---|---|
| 18 | $CH_2CH_2OCH_3$ | H | 160-2 | Calc. C 57.8; H 3.9; N 9.6<br>Found C 58.5; H 4.2; N 10.7 |
| 19 | $CH_2CH_2OCH_3$ | Cl | 208-210 | Calc. C 51.7; H 3.1; N 8.6<br>Found C 51.1; H 3.3; N 8.7 |
| 20 | $CH_2CH_2CN$ | H | 246-8 | Calc. C 58.8; H 2.8: N 14.7<br>Found C 58.4; H 2.8; N 14.8 |
| 21 | $CH_2CH_2CN$ | Cl | 275-6 | Calc C 52.5; H 2.2; N 13.1<br>Found C 53.1; H 2.4; N 13.3 |
| 22 | Cyclopropyl | H | 207-210 | Calc. C 61.7; H 3.3; N 10.3<br>Found C 60.2; H 3.4; N 10.2 |

TABLE 1 continued...

| Ex. No. | $R_1$ | $R_2$ | M.Pt. °C | Analysis |
|---|---|---|---|---|
| 23 | Cyclopropyl | Cl | 250-1 | Calc. C 54.7; H 2.6; N 9.1<br>Found C 54.1; H 2.8; N 9.2 |
| 24 | 4-F cyclohexyl | H | 250-2 | Calc. C 62.5; H 2.5; N 8.6<br>Found C 61.7; H 2.7; N 8.9 |
| 25 | 4-F cyclohexyl | Cl | 298-9 | Calc. C 56.5; H 1.9; N 7.7<br>Found C 56.3; H 2.1; N 8.7 |
| 26 | 4-Cl cyclohexyl | H | 228-230 | Calc. C 59.5; H 2.3; N 8.2<br>Found C 59.2; H 2.3; N 8.4 |
| 27 | 4-Cl cyclohexyl | Cl | 248-250 | Calc. C 54.0; H 1.9; N 7.4<br>Found C 54.3; H 2.7; N 7.3 |

TABLE 1 continued...

| Ex. No. | $R_1$ | $R_2$ | M.Pt.°C | Analysis |
|---|---|---|---|---|
| 28 | $CH(CH_3)COOC_2H_5$ | H | 125-6 | Calc. C 58.9; H 4.3; N 8.1 |
| | | | | Found C 57.3; H 4.2; N 7.7 |
| 29 | $CH(CH_3)COOC_2H_5$ | Cl | 146-8 | Calc. C 54.5; H 3.7; N 7.3 |
| | | | | Found C 51.5; H 6.3; N 8.1 |
| 30 | $C_2H_5$ | Cl | 270-272 | Calc. C 52.9; H 2.7; N 9.5 |
| | | | | Found C 52.7; H 2.7, N 9.4 |
| 31 | $CH_3$ | Cl | 290 (dec) | Calc. C 51.3; H 2.2; N 10.0 |
| | | | | Found C 50.7; H 3.0; N 11.0 |
| 32 | $CH_2CH_2$Phenyl | H | 199-201 | Calc. C 67.8; H 3.9; N 8.3 |
| | | | | Found C 67.9; H 4.1; N 8.8 |
| 33 | $(CH_2)_5CN$ | H | 153-5 | Calc. C 62.3; H 4.3; N 12.8 |
| | | | | Found C 61.3; H 4.7; N 13.4 |
| 34 | $CH(CH_3)CH_2OCH_3$ | H | 134-6 | Calc. C 59.1; H 4.3; N 9.2 |
| | | | | Found C 57.6; H 4.6; N 9.8 |

Example 32

The fungicidal activity of compounds of the invention was investigated by means of the following tests.

(a) Direct protectant activity against vine downy mildew (Plasmopara viticola ; P.v.p)

The test is a direct protectant one using a foliar spray. The lower surfaces of leaves of whole vine plants (cv Cabernet Sauvignon) are sprayed with a solution of active material in 1 :1 water/acetone containing 0.04% "Triton X-155" (trade mark). The spraying is carried out with a moving track sprayer which delivers 620 l/ha, and the concentration of active material is calculated to give an application rate of 1kg/ha. After a subsequent 24 hours under normal glasshouse conditions the lower surfaces of the leaves are inoculated by spraying with an aqueous solution containing $10^4$ zoosporangia/ml. The inoculated plants are kept for 24 hours in a high humidity compartment, 5 days under normal glasshouse conditions and then returned

for a further 24 hours to high humidity. Assessment is based on the percentage of leaf area covered by sporulation compared with that on control leaves.

b) Activity against wheat leafspot (Leptosphaeria nodorum ; Ln.)

The test is a direct therapeutic one, using a foliar spray. Leaves of wheat plants (cv Mardler), at the single leaf stage, are inoculated by spraying with an aqueous suspension containing $1 \times 10^6$ spores/ml. The inoculated plants are kept for 24 hours in a high humidity compartment prior to treatment. The plants are sprayed with a solution of the test compound at a dosage of 1 kilogram of active material per hectare using a track sprayer as described under (a). After drying, the plants are kept for 6-8 days at 20-25°C and moderate humidity, followed by assessment. Assessment is based on the density of lesions per leaf compared with that on leaves of control plants.

c)Activity against barley powdery mildew (Erysiphe graminis f.sp. hordei ; Eg)

The test is a direct therapeutic one, using a foliar spray. Leaves of barley seedlings, (cv. Golden Promise) are inoculated by dusting with mildew conidia one day prior to treatment with the test compound. The inoculated plants are kept overnight at glasshouse ambient temperature and humidity prior to treatment. The plants are sprayed with the test compound at a dosage of 1 kilogram of active material per hectare using a track sprayer as described under (a). After drying, plants are returned to a compartment at 20-25°C and moderate humidity for up to 7 days, followed by assessment. Assessment is based on the percentage of leaf area covered by sporulation compared with that on leaves of control plants.

d) Activity against rice leaf blast (Pyricularia oryzae Po)

The test is a direct therapeutic one using a foliar spray. The leaves of rice seedlings (about 30 seedlings per pot) are sprayed with an aqueous suspension containing $10^5$ spores/ml 20-24 hours prior to treatment with the test compound. The inoculated plants are kept overnight in high humidity and then allowed to dry before spraying with the test compound at a dosage of 1 kilogram of active material per hectare using a track sprayer as described under (a). After treatment the plants are kept in a rice compartment at 25-30°C and high humidity. Assessments are made 4-5 days after treatment and are based on the density of necrotic lesions per leaf when compared with control plants.

e) Activity against tomato early blight (Alternaria solani ; As)

This test measures the contact prophylactic activity of test compounds applied as a foliar spray.

Tomato seedlings (cv Outdoor Girl) are grown to the stage at which the second true leaf is expanded. The plants are treated using a track sprayer as described under (a). Test compounds are applied as solutions or suspensions in a mixture of acetone and water (50 :50v/v) containing 0.04% surfactant ("TWEEN 20" - Trademark).

One day after treatment the seedlings are inoculated by spraying the leaf upper surfaces with a suspension of A. solani conidia containing $10^4$ spores/ml. For 3 days after inoculation plants are kept moist in a glasshouse compartment at or near 100% RH and 21°C. Thereafter plants are kept under humid, but not saturated, conditions.

Disease is assessed 7 days after inoculation, based on the density and spread of lesions.

f) Activity against wheat eyespot in-vitro (Pseudocercosporella herpotrichoides ; Phl)

This test measures the in vitro activity of compounds against the fungus causing wheat eyespot.

The Test Compound is dissolved or suspended in acetone and is added to molten half strength Potato Dextrose Agar to give a final concentration of 100ppm compound and 3.5% acetone. After agar has set, plates are inoculated with 6mm diameter plugs of agar/mycelium taken from a 14 day old culture of P. herpotrichoides.

Plates are incubated at 20°C for 12 days and radial growth from the inoculation plug is measured.

The extent of disease control in the above test is expressed as a rating compared with a diluent-sprayed-control according to the criteria :-

0 = less than 50% disease control
1 = about 50-80% disease control

2 = greater than 80%
The results of these tests are given in Table 2 below :

Table 2

| Compound of Ex. No. | Fungicidal Rating | | | | | |
|---|---|---|---|---|---|---|
| | Pvp. | Ph. | As. | Po. | Ln | Eg. |
| 1 | 2 | | | | | |
| 3 | 1 | 1 | | | | |
| 4 | 1 | | | 2 | | |
| 5 | 2 | | | 1 | | |
| 7 | | | | 2 | | |
| 8 | | | | 2 | | |
| 9 | 2 | | | 1 | | |
| 10 | 2 | | | | | |
| 11 | 1 | | | | | |
| 12 | 2 | | 1 | | | |
| 13 | | | 1 | | 1 | |
| 14 | 2 | | 2 | | 1 | |
| 15 | 2 | | | | 1 | |
| 16 | 2 | | | | | 1 |
| 17 | 2 | | | | | 1 |
| 18 | 2 | | 2 | | | |
| 19 | 2 | | | | | |
| 20 | 2 | | | | | |
| 21 | 2 | | | | | |
| 22 | 2 | | 2 | | | |
| 23 | 1 | | 1 | | | 1 |
| 24 | 2 | | | | | |
| 26 | 1 | | | | | |
| 28 | 2 | | | | | |
| 29 | 2 | | | | | |
| 30 | 2 | | 1 | | | |

**Claims**

1. A fungicidal composition which comprises a carrier and, as active ingredient, a naphthimidazolone of the formula :

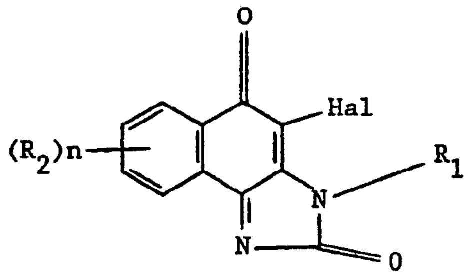

II

wherein Hal represents a halogen atom ; $R_1$ represents a hydrogen atom, an alkyl, alkenyl or alkynyl group of to up 14 carbon atoms, a cycloalkyl group of 3 to 10 carbon atoms, a benzyl group or a phenyl group, each group being optionally substituted by one or more substituents selected from halogen atoms, nitro, cyano, hydroxyl, $C_{1-6}$ alkyl, $C_{1-6}$ haloalkyl, $C_{1-6}$ alkoxy and $C_{1-6}$ alkoxycarbonyl groups ; $R_2$ represents a halogen atom or an alkyl or haloalkyl group of 1 to 14 carbon atoms ; and n is 0, 1 or 2.

2. A composition as claimed in claim 1 wherein the active ingredient is a compound of formula II wherein Hal represents a fluorine, chlorine, or bromine atom ; $R_1$ represents a hydrogen atom, an alkyl group of 1 to 14 carbon atoms optionally substituted by a fluorine or chlorine atom, or a cyano, alkoxy or alkoxycarbonyl group, an alkenyl or alkynyl group of up to 6 carbon atoms, a cycloalkyl group of 3 to 8 carbon atoms optionally substituted by a fluorine or chlorine atom, a benzyl group or a phenyl group ; $R_2$ represents a chlorine atom or an alkyl group of 1 to 6 carbon atoms and n is 0 or 1.

3. A composition as claimed in claim 2 wherein Hal represents a chlorine atom ; $R_1$ represents a methyl, ethyl, propyl, butyl or tridecyl group optionally substituted by a fluorine or chlorine atom or a cyano, methoxy or ethoxycarbonyl group, a propenyl or propynyl group, a cyclopropyl or cyclohexyl group optionally substituted by a fluorine or chlorine atom, or a benzyl or phenyl group ; and $R_2$ is a chlorine atom or a methyl group.

4. A composition as claimed in claim 1, 2 or 3 which comprises at least two carriers, at least one of which is a surfaceactive agent.

5. A method for making a fungicidal composition as defined in claim 1 which comprises bringing a naphthimidazolone as defined in claim 1 into association with at least one carrier

6. A naphthimidazolone of the formula II as shown in claim 1, wherein Hal, $R_1$, $R_2$ and n are as defined in claim 1, subject to the provisos that, when Hal is a chlorine atom and n=0, then $R_1$ is not a hydrogen atom or a phenyl group, and, when Hal is a bromine atom and n=0, then $R_1$ is not a phenyl group.

7. A process for the preparation of a compound as defined in claim 6, which comprises heating chlorosulphonylisocyanate with an amino naphthoquinone of formula III :

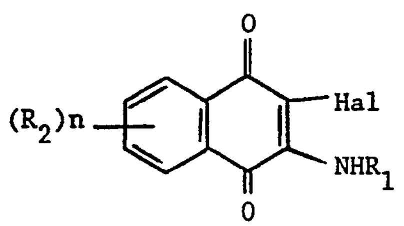

III

the terms Hal, $R_1$, $R_2$ and n having the meanings defined in claim 6

8. A naphthimidazolone as defined in claim 1, whenever prepared by a process as claimed in claim 7.

9. A method of combating fungus at a locus, characterised by treating the locus with a fungicidally effective amount of a composition as claimed in any one of claims 1-4 or a compound as claimed in claim 6 or 8.

10. A method as claimed in claim 9, wherein the locus comprises plants subject to or subjected to fungal attack, seeds of such plants, or the medium in which the plants are growing or are to be grown.

11. The use as a fungicide of a compound of formula II as defined in any one of claims 1-6 or 8.

**Ansprüche**

1. Fungizide Zusammensetzung, umfassend einen Träger und als wirksamen Bestandteil ein Naphthimidazolon mit der Formel :

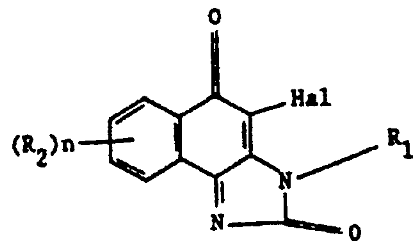

(II),

worin

Hal ein Halogenatom darstellt ;

$R_1$ ein Wasserstoffatom, eine Alkyl-, Alkenyl- oder Alkinylgruppe mit bis zu 14 Kohlenstoffatomen, eine Cycloalkylgruppe mit 3 bis 10 Kohlenstoffatomen, eine Benzylgruppe oder eine Phenylgruppe darstellt, wobeijede Gruppe gegebenenfalls durch einen oder mehrere Substituenten, ausgewählt unter Halogenatomen, Nitro-, Cyano, Hydroxyl-, $C_{1-6}$ Alkyl-, $C_{1-6}$ Halogenalkyl, $C_{1-6}$ Alkoxycarbonylgruppen, substituiert ist ;

$R_2$ ein Halogenatom oder eine Alkyl- oder Halogenalkylgruppe mit 1 bis 14 Kohlenstoffatomen darstellt; und

n den Wert 0, 1 oder 2 hat.

2. Zusammensetzung nach Anspruch 1, worin der wirksame Bestandteil eine Verbindung der Formel II ist, worin

Hal ein Fluor-, Chlor- oder Bromatom ;

$R_1$ ein Wasserstoffatom, eine gegebenenfalls durch ein Fluor- oder Chloratom substituierte Alkylgruppe mit 1 bis 14 Kohlenstoffatomen oder eine cyano-, Alkoxy- oder Alkoxycarbonylgruppe, eine Alkenyl- oder Alkinylgruppe mit bis zu 6 Kohlenstoffatomen, eine gegebenenfalls durch ein Fluor- oder Chloratom substituierte Cycloalkylgruppe mit 3 bis 8 Kohlenstoffatomen, eine Benzylgruppe oder eine Phenylgruppe darstellt ;

$R_2$ ein Chloratom oder eine Alkylgruppe mit 1 bis 6 Kohlenstoffatomen bedeutet und

n den Wert 0 oder 1 hat.

3. Zusammensetzung nach Anspruch 2, worin

Hal ein Chloratom bedeutet ;

$R_1$ eine Methyl-, Ethyl-, Propyl-, Butyl- oder Tridecylgruppe, die gegebenenfalls durch ein Fluor- oder Chloratom substituiert ist, oder eine Cyano-, Methoxy- oder Ethoxycarbonylgruppe, eine Propenylgruppe oder Propinylgruppe, eine gegebenenfalls durch ein Fluor- oder Chloratom substituierte Cyclopropyl- oder Cyclohexylgruppe oder eine Benzyl- oder Phenylgruppe darstellt ; und

$R_2$ ein Chloratom oder eine Methylgruppe bedeutet.

4. Zusammensetzung nach Anspruch 1, 2 oder 3, welche wenigstens zwei Träger umfaßt, von denen wenigstens einer ein oberflächenaktives Mittel ist.

5. Verfahren zur Bereitung einer fungiziden Zusammensetzung, wie in Anspruch 1 definiert, welches ein Zusammenbringen eines Naphthimidazolons, wie in Anspruch 1 definiert, mit wenigstens einem Träger umfaßt.

6. Ein Naphthimidazolon der Formel II, wie in Anspruch 1 dargestell, worin Hal, $R_1$, $R_2$ und n wie in Anspruch 1 definiert sind, mit den Maßgaben, daß dann, wenn Hal ein Chloratom bedeutet und n den Wert 0 hat, $R_1$ nicht ein Wasserstoffatom oder eine Phenylgruppe bedeutet, und daß dann, wenn Hal ein Bromatom ist und n den Wert 0 hat, $R_1$ nicht eine Phenylgruppe bedeutet.

7. Verfahren zur Herstellung einer Verbindung, wie in Anspruch 6 definiert, welches ein Erwärmen von Chlorsulfonylisocyanat mit einem Aminonaphthochinon der Formel III :

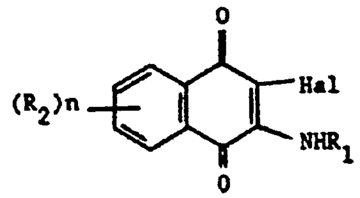

(III)

umfaßt, wobei die Begriffe Hal, $R_1$, $R_2$ und n die in Anspruch 6 definierten Bedeutungen aufweisen.

8. Naphthimidazolon gemäß Definition in Anspruch 1, wann immer es nach einem Verfahren gemäß Anspruch 7 hergestellt ist.

9. Verfahren zur Bekämpfung von Pilzen an einem Ort, gekennzeichnet,durch Behandeln des Ortes mit einer fungizid wirksamen Menge einer Zusammensetzung, wie in einem der Ansprüche 1 bis 4 beansprucht, oder mit einer Verbindung, wie in Anspruch 6 oder 8 beansprucht.

10.Verfahren nach Anspruch 9, worin der Ort Pflanzen, die einem Pilzbefall unterliegen oder in Zukunft unterliegen, Sämlinge und Samen solcher Pflanzen oder das Medium umfaßt, worin die Pflanzen wachsen oder gezogen werden sollen.

11. Verwendung einer Verbindung der Formel II, wie in einem der Ansprüche 1 bis 6 oder 8 definiert, als Fungizid.

**Revendications**

1. Une composition fongicide qui comprend un support et, comme ingrédient actif, une naphtimidazolone de formule :

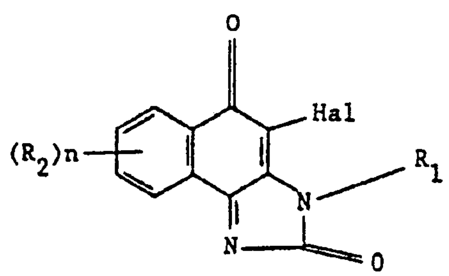

II

dans laquelle Hal représente un atome d'halogène ; $R_1$ représente un atome d'hydrogène, un groupe alkyle, alcényle ou alcynyle de jusqu'à 14 atomes de carbone, un groupe cycloalkyle de 3 à 10 atomes de carbone, un groupe benzyle ou un groupe phényle, chaque groupe étant facultativement substitué par un ou plus d'un substituants choisis parmi les atomes d'halogène, les groupes nitro, cyano, hydroxyle, alkyle en $C_1$ à $C_6$, haloalkyle en $C_1$ à $C_6$ alcoxy $C_1$ à $C_6$et alcoxycarbonyle en $C_1$ à $C_6$ ; $R_2$ représente un atome d'halogène ou un groupe alkyle ou haloalkyle de 1 à 14 atomes de carbone ; et n est 0, 1 ou 2.

2. Une composition telle que revendiquée dans la revendication 1, dans laquelle l'ingrédient actif est un composé de formule II dans laquelle Hal représente un atome de fluor, de chlore ou de brome, $R_1$ représente un atome d'hydrogène, un groupe alkyle de 1 à 14 atomes de carbone facultativement substitué par un atome de fluor ou de chlore, ou bien un groupe cyano, alcoxy ou alcoxycarbonyle, un groupe alcényle ou alcynyle de jusqu'à 6 atomes de carbone, un groupe cycloalkyle de 3 à 8 atomes de carbone, facultativement substitué par un atome de fluor ou de chlore, un groupe benzyle ou un groupe phényle ; $R_2$ représente un atome de chlore ou un groupe alkyle de 1 à 6 atomes de carbone et n est 0 ou 1.

3. Une composition telle que revendiquée dans la revendication 2, dans laquelle Hal représente un atome de chlore ; $R_1$ représente un groupe méthyle, éthyle, propyle, butyle ou tridécyle, facultativement substitué par un atome de fluor ou de chlore, ou bien un groupe cyano, méthoxy ou éthoxycarbonyle, un groupe propényle ou propynyle, un groupe cyclopropyle ou cyclohexyle facultativement substitué par un atome de fluor ou de chlore, ou bien un groupe benzyle et phényle ; et R2 est un atome de chlore ou un

groupe méthyle.

4. Une composition telle que revendiquée dans la revendication 1, 2 ou 3, qui comprend au moins deux supports, dont au moins un est un agent tensio-actif.

5. Une méthode pour préparer une composition fongicide telle que définie dans la revendication 1, qui consiste à mettre une naphtidimazolone tel que définie dans la revendication 1 en association avec au moins un support.

6. Une naphtimidazolone de formule II telle que représentée dans la Figure 1, dans laquelle Hal, $R_1$, $R_2$ et n sont tels que définis dans la revendication 1, soumis aux conditions que, si Hal est un atome de chlore et n=0, alors $R_1$ n'est pas un atome d'hydrogène ni un groupe phényle, et, si Hal est un atome de brome et n=0, alors $R_1$ n'est pas un groupe phényle.

7. Un procédé pour la préparation d'un composé tel que défini dans la revendication 6, qui consiste à chauffer un isocyanate de chlorosulfonyle avec une amino naphtoquinone de formule III :

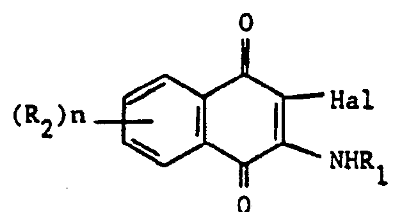

III

les références Hal, $R_1$, $R_2$ et n ayant les significations spécifiées dans la revendication 6.

8. Une naphtimidazolone telle que définie dans la revendication 1, lorsqu'elle est préparée par un procédé tel que revendiqué dans la revendication 7.

9. Une méthode pour combattre les moisissures en un endroit, caractérisée par le traitement de l'endroit avec une quantité efficace du point de vue fongicide d'une composition telle que revendiquée dans l'une quelconque des revendications 1 à 4 ou un composé tel que défini dans la revendication 6 ou 8.

10. Une méthode telle que revendiquée dans la revendication 9, dans laquelle l'endroit comporte des plantes sujettes à ou soumises à des attaques fongiques, des graines de ces plantes, ou bien le milieu dans lequel les plantes croissent ou doivent être cultivées.

11. L'utilisation comme fongicide d'un composé de formule II tel que défini dans l'une quelconque des revendications 1 à 6 ou 8.